# EUROPÄISCHE PATENTSCHRIFT

(11) **EP 0 690 858 B1**
(45) Veröffentlichungstag und Bekanntmachung des Hinweises auf die Patenterteilung: **24.06.1998**
(21) Anmeldenummer: 94911153.8
(22) Anmeldetag: 11.03.1994
(51) Int. Cl.: C07D 417/06, A01N 43/40, C07D 213/85, C07D 213/64, C07D 401/06, A01N 43/54, A01N 43/78

(54) **SUBSTITUIERTE STICKSTOFF-HETEROCYCLEN UND IHRE VERWENDUNG ALS SCHÄDLINGSBEKÄMPFUNGSMITTEL**
SUBSTITUTED NITROGEN HETEROCYCLES AND THEIR USE AS PESTICIDES
HETEROCYCLES D'AZOTE SUBSTITUES ET LEUR UTILISATION COMME PESTICIDES

(30) Priorität: 24.03.1993 DE 4309552
(43) Veröffentlichungstag der Anmeldung: 10.01.1996
(73) Patentinhaber: BAYER AG, 51368 Leverkusen (DE)
(72) Erfinder: KANELLAKOPULOS, Johannes, D-41542 Dormagen (DE); FUCHS, Rainer, D-42113 Wuppertal (DE); JANSEN, Johannes, Rudolf, D-40789 Monheim (DE); SCHINDLER, Michael, D-51467 Bergisch Gladbach (DE); ERDELEN, Christoph, D-40799 Leichlingen (DE); LEICHT, Wolfgang, D-51371 Leverkusen (DE); WACHENDORFF-NEUMANN, Ulrike, D-40789 Monheim (DE); TURBERG, Andreas, D-40699 Erkrath (DE)
(86) Internationale Anmeldenummer: EP9400757
(87) Internationale Veröffentlichungsnummer: WO9421632

(56) Entgegenhaltungen:
- EP-A- 0 272 824
- EP-A- 0 344 500
- EP-A- 0 357 201
- EP-A- 0 367 410
- EP-A- 0 402 717
- EP-A- 0 500 297
- EP-A- 0 530 702
- WO-A-90/05134
- DE-A- 2 637 477
- US-A- 4 028 084
- TETRAHEDRON, (INCL. TETRAHEDRON REPORTS) Bd. 35 , OXFORD GB Seiten 2591 - 2593 F.M. MORACCI ET AL. 'Reactivity of pseudobases from pyridinium salts competition between hydrogen transfer and ring-opening reactions'
- CHEMICAL ABSTRACTS, vol. 113, no. 1, 2. Juli 1990, Columbus, Ohio, US; abstract no. 185r, V.G. KULNEVICH ET AL. 'Synthesis and antiviral activity of N-alkyl-3-cyano-2-pyridones and 3-cyano-2-alkoxypyridines' Seite 179 ;

## Beschreibung

Die vorliegende Erfindung betrifft neue substituierte Stickstoff-Heterocyclen, Verfahren zu ihrer Herstellung und ihre Verwendung als Schädlingsbekämpfungsmittel.

Substituierte Stickstoffheterocyclen und ihre Verwendung als Insektizide sind bekannt aus z.B. EP-OS 259 738, 367 410 und 402 717. Die Wirkung der bekannten Verbindungen ist jedoch nicht immer zufriedenstellend.

Es wurden neue substituierte Stickstoff-Heterocyclen der allgemeinen Formel (I), 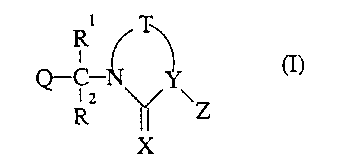 gefunden,
in welcher die Gruppierung 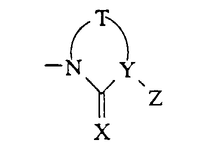 eine der folgenden Bedeutungen 1 bis 14 annimmt: 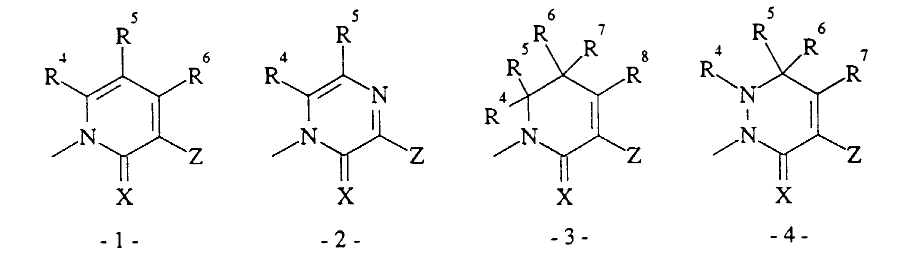 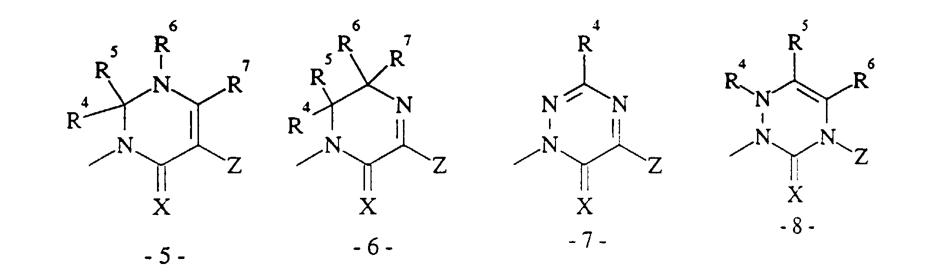 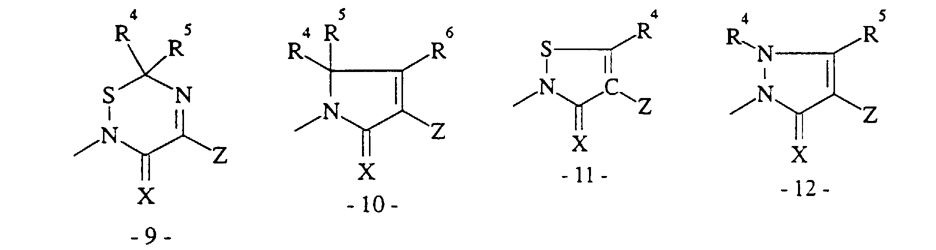 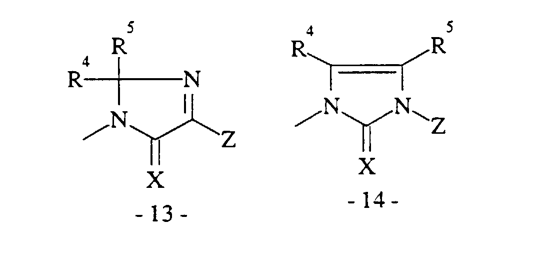 wobei
- R¹: für Wasserstoff, Methyl oder Ethyl steht,
- R²: für Wasserstoff steht,
- X: für Sauerstoff, Schwefel oder NR³ steht,
- Z: für eine Cyano- oder Nitrogruppe steht,
- Q: eine der folgenden Bedeutungen annimmt: 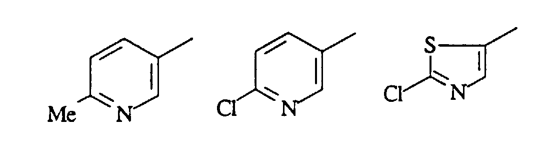 in welcher
- R³: für Wasserstoff oder Methyl steht,
- R⁴, R⁵, R⁶, R⁷ und R⁸: unabhängig voneinander für Wasserstoff, Fluor, Chlor, Brom, C₁₋₂-Alkyl, C₁₋₂-Alkoxy und gegebenenfalls durch Fluor, Chlor, Brom, C₁₋₂-Alkyl, Trifluormethyl, C₁₋₂-Alkoxy, Trifluormethoxy ein- oder mehrfach substituiertes Phenyl steht.

Weiterhin wurde gefunden, daß sich die neuen substituierten Stickstoff-Heterocyclen der Formel (I) durch hervorragende insektizide Wirkungen auszeichnen.

Weiterhin wurde gefunden, daß man die neuen, substituierten Stickstoff-Heterocyclen der allgemeinen Formel (I), 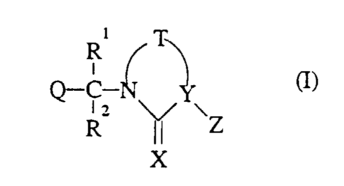 in welcher die Reste die oben angegebene Bedeutung besitzen erhält, wenn man Stickstoff-Heterocyclen der Formel (II) 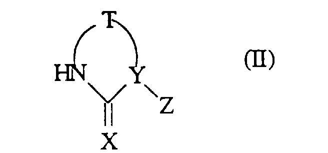 in welcher
T, X, Y und Z die oben angegebene Bedeutung haben,
mit Halogenmethyl-Verbindungen der Fromel (III) 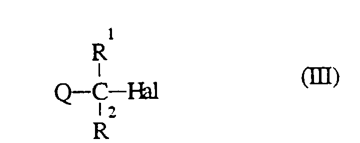 in welcher
- Hal: für Chlor, Brom oder Iod steht, und Q, R¹ und R² die oben angegebene Bedeutung haben,
gegebenenfalls in Gegenwart eines Verdünnungsmittels und in Gegenwart einer Base umsetzt.

Durch Formel (I) werden auch die tautomeren Formen der erfindungsgemäßen Verbindungen erfasst wie z.B.

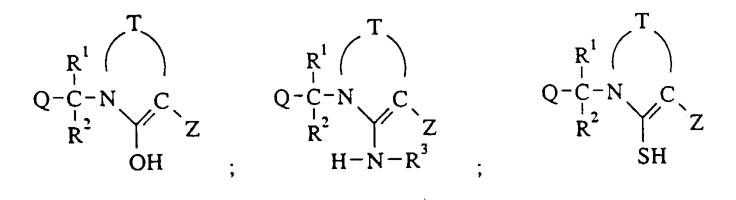

Verwendet man gemäß Verfahren (A) 3-Cyano-2-hydroxypyridin und 2-Chlor-5-chlormethyl-pyridin als Ausgangsprodukte, so kann der Verlauf des erfindungsgemäßen Verfahrens durch folgendes Reaktionsschema wiedergegeben werden: 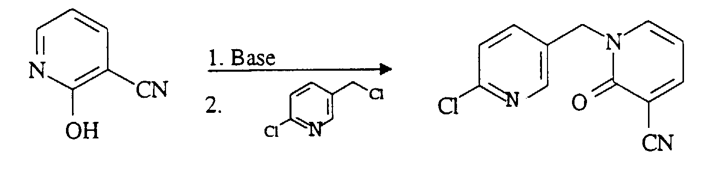 Die bei dem obigen Verfahren als Ausgangsstoff benötigten Verbindungen der Formel (II), 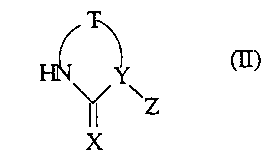 in welcher
T, X, Y und Z die oben angegebenen Bedeutungen haben,
sind bekannt oder lassen sich nach im Prinzip bekannten Methoden herstellen. So erhält man z.B. 3-Cyano-pyridone der Formel (II-1), wenn man 1.3-Diketone der Formel (IV), 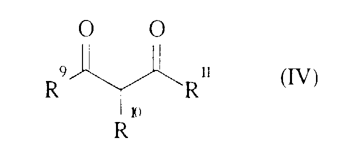 in welcher
- R⁹ und R¹¹: unabhängig voneinander für Wasserstoff oder C₁₋₃-Alkyl steht,
- R¹⁰: für Wasserstoff oder C₁₋₃-Alkyl oder Halogen steht,
mit Cyanessigsäureamid kondensiert (J. Heterocycl. Chem. 19, 1297-1300 (1982)).

Die bei dem obigen Verfahren als Ausgangsstoff benötigten Verbindungen der Formel (III), 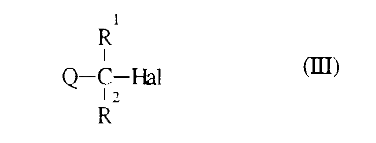 in welcher
Hal, R¹, R² und Q die oben angegebenen Bedeutungen haben, sind bekannt oder sind nach im Prinzip bekannten Methoden erhältlich (JP 48 099 178: DE 4016 175; DE 3 631 538).

Das Verfahren ist dadurch gekennzeichnet, daß man Verbindungen der Formel (II), in welcher T, X, Y und Z die oben angegebenen Bedeutungen haben, mit Verbindungen der Formel (III), in welcher Hal, R¹, R² und Q die oben angegebenen Bedeutungen haben, in Gegenwart von Säureakzeptoren zur Reaktion bringt.

Als Säureakzeptoren können bei dem erfindungsgemäßen Verfahren (a) alle üblicherweise für derartige Umsetzungen verwendbaren Säurebindemittel eingesetzt werden. Vorzugsweise in Frage kommen Alkalimetall- und Erdalkalimetall-hydride, wie Lithium-, Natrium-, Kalium- und Calcium-hydrid, Alkalimetall- und Erdalkalimetallhydroxide, wie Lithium-, Natrium-, Kalium- und Calcium-hydroxid, Alkalimetall- und Erdalkalimetall-carbonate und -hydrogencarbonate, wie Natrium- und Kaliumcarbonat oder Hydrogencarbonat sowie Calciumcarbonat, Alkalimetallacetate, wie Natrium- und Kalium-acetat, Alkalimetallalkoholate, wie Natrium- und Kaliummethylat, -ethylat, -propylat, -isopropylat, -butylat, -isobutylat und tert-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, Ethyldiisopropylamin, Ethyldicyclohexylamin, N,N-Dimethylbenzylamin, N,N-Dimethyl-analin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-Methyl-pyridin, 1,5-Diazabicyclo-[ 4,3,0]-non-5-en (DBN), 1,8-Diazabicyclo-[5,4,0]-undec-7-en (DBU) und 1,4-Diazabicyclo-[2,2,2]-octan (DABCO).

Das erfindungsgemäße Verfahren zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methylpyrrolidon sowie Dimethylsulfoxid, Tetramethylensulfon und Hexamethylphosphorsäuretriamid.

Die Reaktionstemperaturen können bei dem erfindungsgemäßen Verfahren in einem größeren Bereich variiert werden. Im allgemeinen arbeitet man bei Temperaturen zwischen 0°C und 200°C, vorzugsweise bei Temperaturen zwischen 20°C und 150°C.

Das erfindungsgemäße Verfahren wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden (vergleiche die Herstellungsbeispiele).

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.

Aus der Ordnung der Orthoptera z.B. Blatta orientalis, Periplaneta amencana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.
Aus der Ordnung der Isoptera z.B. Reticulitermes spp.

Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothnps femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agnotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.
Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten und Endoparasiten) wie Schildzecken, Lederzecken, Räudemilben, Laufmilben, Fliegen (stechend und leckend), parasitierende Fliegenlarven, Läuse, Haarlinge, Federlinge, Flöhe und endoparasitisch lebende Würmer.

Sie sind gegen normalsensible und resistente Arten und Stämme sowie gegen alle parasitierenden und nichtparasitierenden Entwicklungsstadien der Ekto- und Endoparasiten wirksam.

Die eindungsgemäßen Wirkstoffe zeichnen sich durch eine hohe insektizide und akarizide Wirksamkeit aus.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Fenstverkapselungen in polymeren Stoffen

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate: als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel: als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Ole sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die gute Pflanzenverträglichkeit der Wirkstoffe in den zur Bekämpfung von Schädlingen notwendigen Konzentrationen erlaubt eine Behandlung von oberirdischen Pflanzenteilen, von Pflanz- und Saatgut, und des Bodens.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen. Spritzen, Sprühen, Streuen, Behandlung des Saatguts.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10kg Wirkstoff pro Hektar Bodenfläche, vorzugsweise zwischen 0,05 und 5 kg pro ha.

### Beispiel A

### Phaedon-Larven-Test

- Lösungsmittel :: 7 Gewichtsteile Dimethylformamid
- Emulgator :: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit werden die Pflanzen mit Meerrettichblattkäfer-Larven (Phaedon cochleariae) besetzt. Nach jeweils 3 Tagen wird die Abtötung in % bestimmt. Dabei bedeutet 100 %, daß alle Käfer-Larven abgetötet wurden; 0 % bedeutet, daß keine Käfer-Larven abgetötet wurden.

Bei diesem Test zeigen z.B. die Verbindungen gemäß Herstellungsbeispiele überlegene Wirksamkeit gegenüber dem Stand der Technik: 1,2.

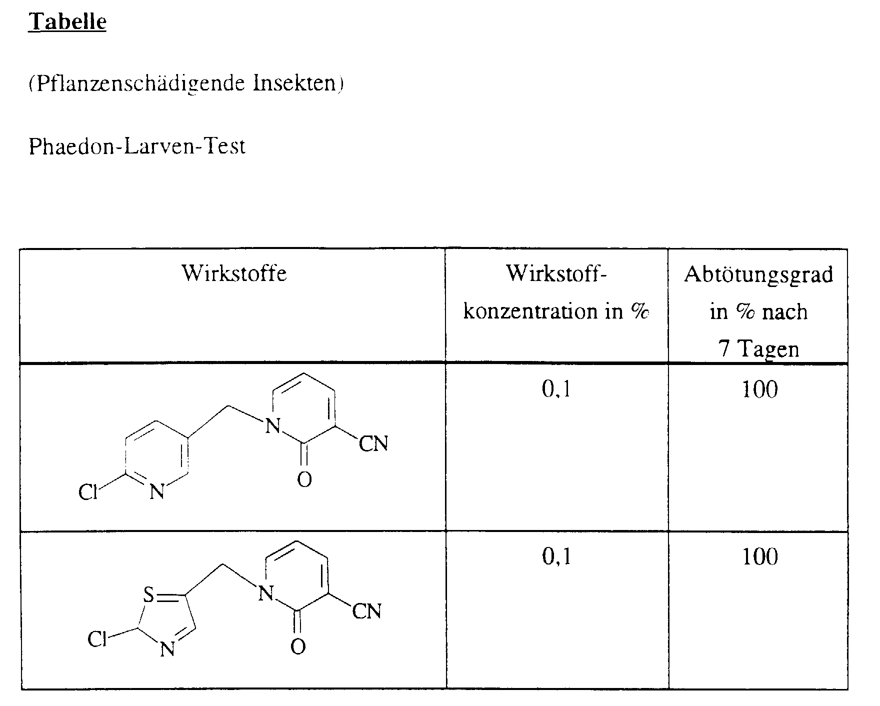

### Beispiel B

### Plutella-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0% bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: 1, 2.

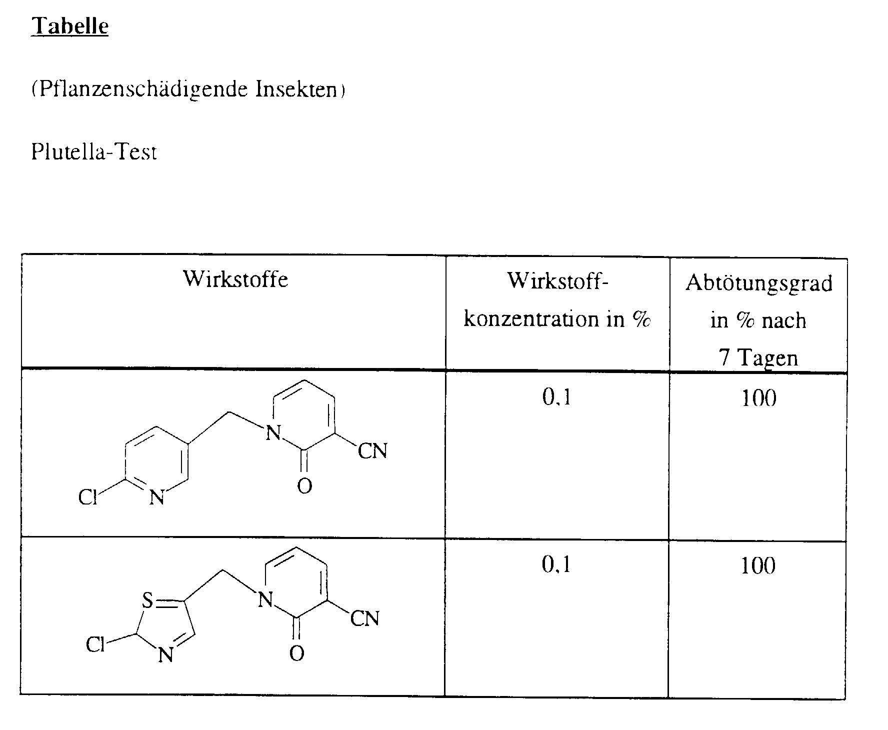

### Beispiel C

### Nephotettix - Test

- Lösungsmittel:: 3 Gewichtsteile Dimethylformamid
- Emulgator:: 1 Gewichtsteil Alkylarylpolyglakolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit Larven der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

Bei diesem Test zeigen z.B. die folgenden Verbindungen der Herstellungsbeispiele überlegene Wirksamkeit gegenüber dem Stand der Technik: 1,2.

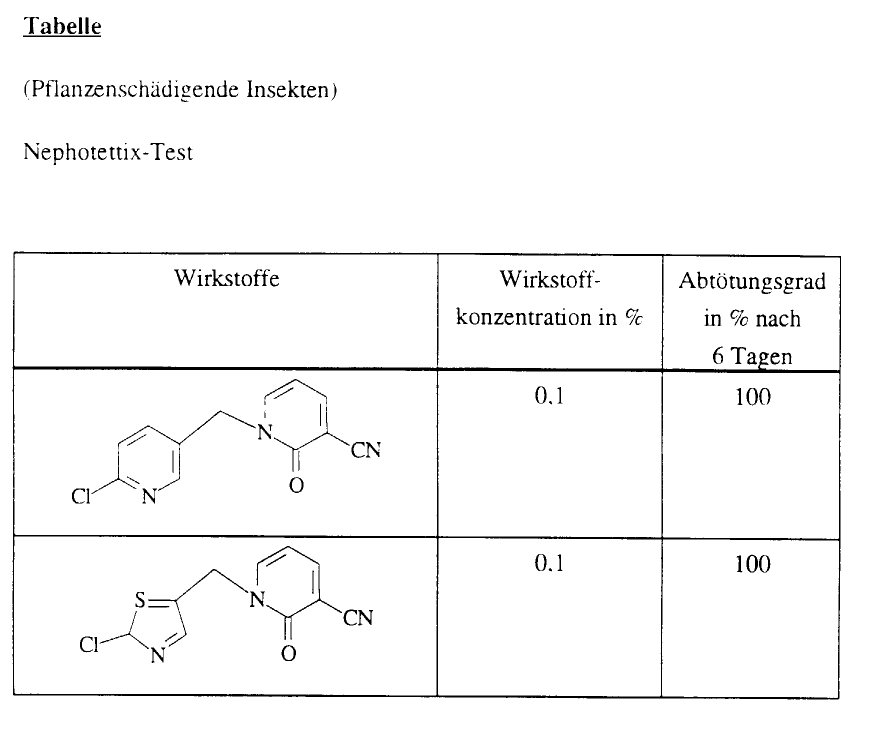

### Beispiel D

### Myzus - Test

- Lösungsmittel:: 31 Gewichtsteile Aceton
- Emulgator:: 1 Gewichtsteil Alkylarylpolyglakolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschte Konzentration.

Keimlinge der Dicken Bohne (Vicia faba), welche mit der Grünen Pfirsichblattlaus (Myzus persicae) befallen sind, werden in die Wirkstoffzubereitung der gewünschten Konzentration getaucht und in eine Plastikdose gelegt.

Nach der gewünschten Zeit wird die Abtötung in % bestimmt. Dabei bedeutet 100 %, daß alle Tiere abgetötet wurden; 0 % bedeutet, daß keine Tiere abgetötet wurden.

Bei diesem Test zeigen z.B. die folgenden Verbindungen der Herstellungsbeispiele überlegene Wirksamkeit gegenüber dem Stand der Technik: 1.

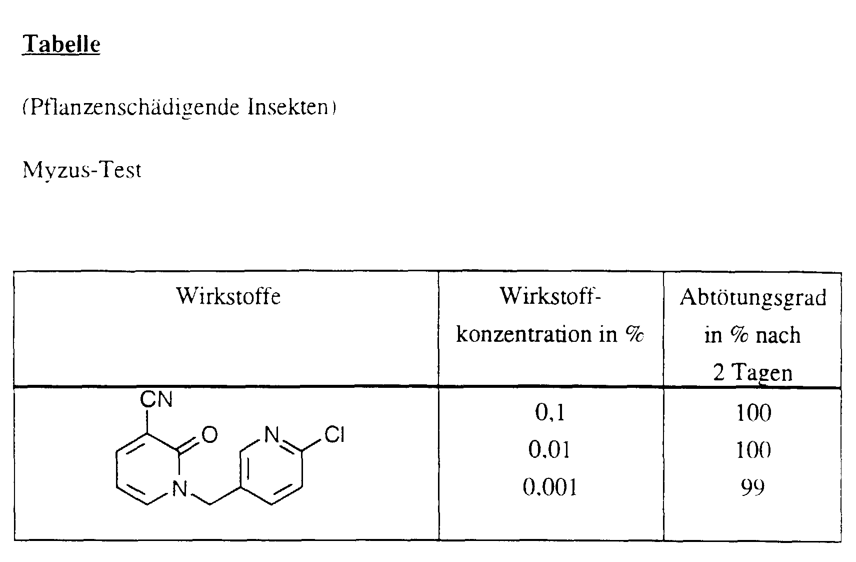

### Herstellungsbeispiele

### Beispiel 1

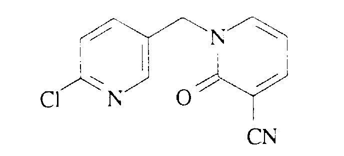

2,0 g (0,017 Mol) 3-Cyano-2-pyridon (Braña, Rodriguez, J. Heterocycl. Chem., 1297 (1982)) werden in 30 ml abs. N,N-Dimethylformamid gelöst und bei 0°C mit 0.56 g (0.019 Mol) Natriumhydrid (80 %ige Suspension) versetzt. Nach 20minütigem Rühren tropft man eine Lösung von 2,75 g (0.017 Mol) 2-Chlor-5-Chlormethylpyridin in 10ml abs. Acetonitril zu und rührt 3 Stunden bei Raumtemperatur. Das Reaktionsgemisch wird dann auf 400 ml Wasser gegeben, mit Ethylacetat extrahiert und die organische Phase nach Trocknen über Magnesiumsulfat zur Trockene destilliert. Der Rückstand wird mit wenig Diethylether verrührt und abgesaugt. Man erhöht auf diese Weise 3,2 g 1-(2'-Chlorpyridin-3-yl)-3-cyano-2-pyridon vom Schmelzpunkt 145°C.

Analog wurden erhalten:

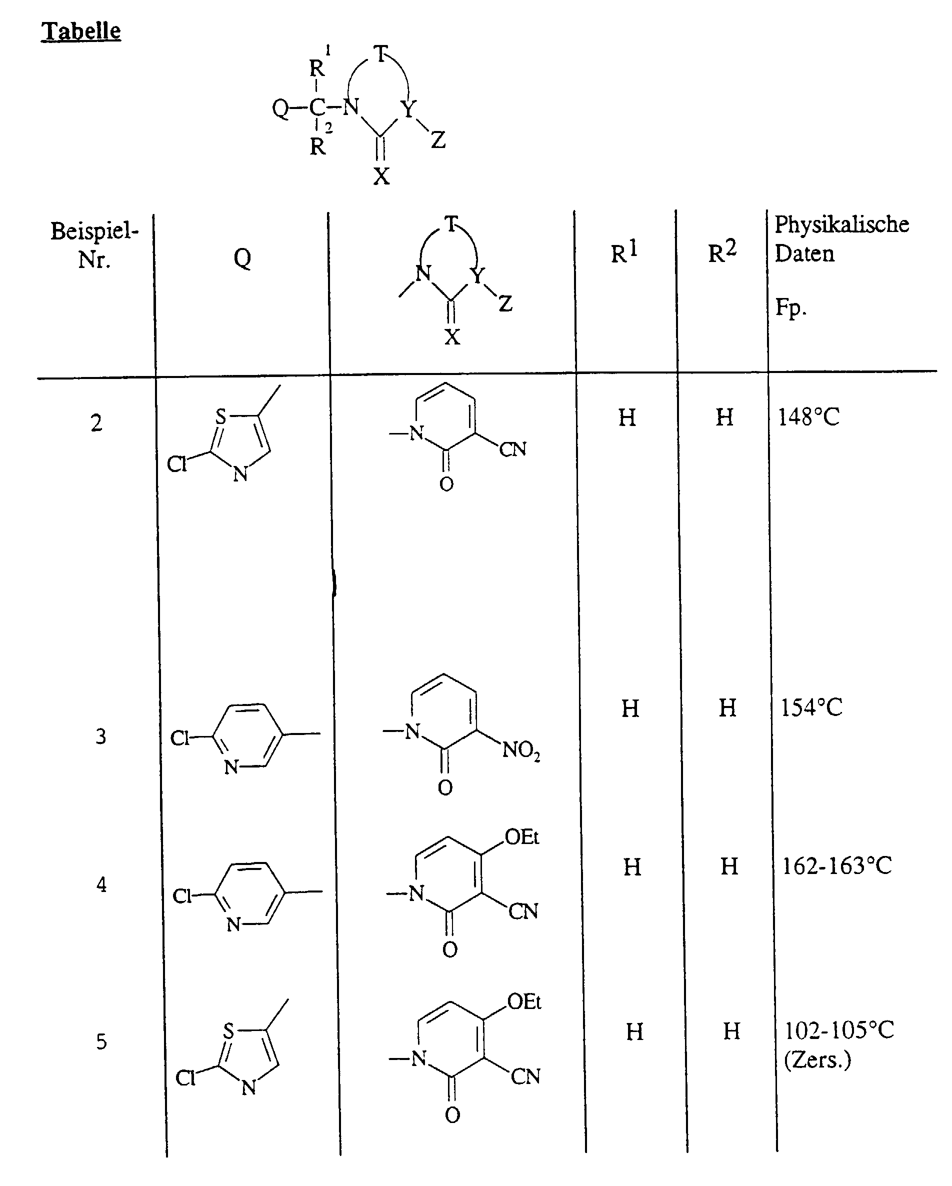

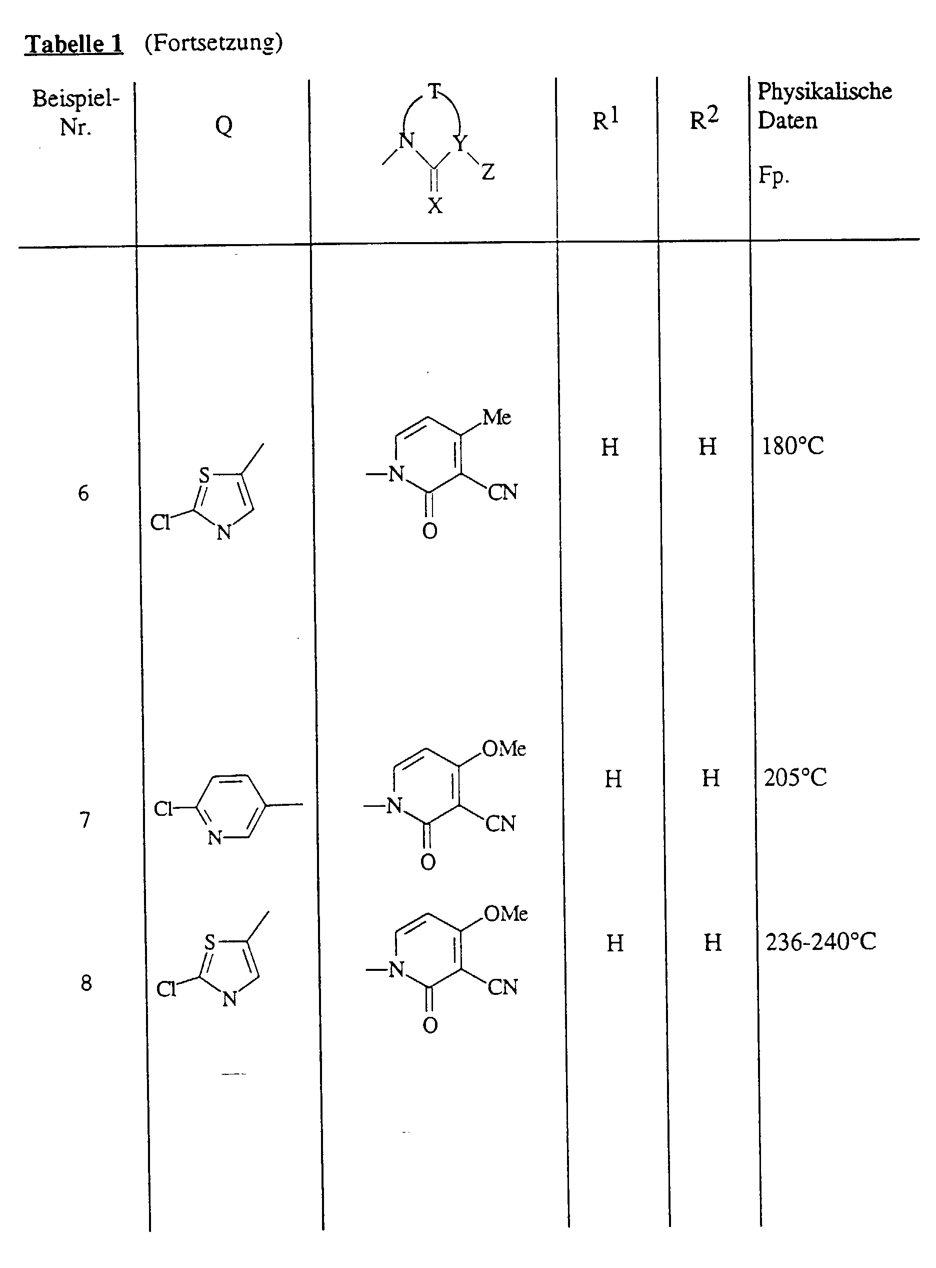

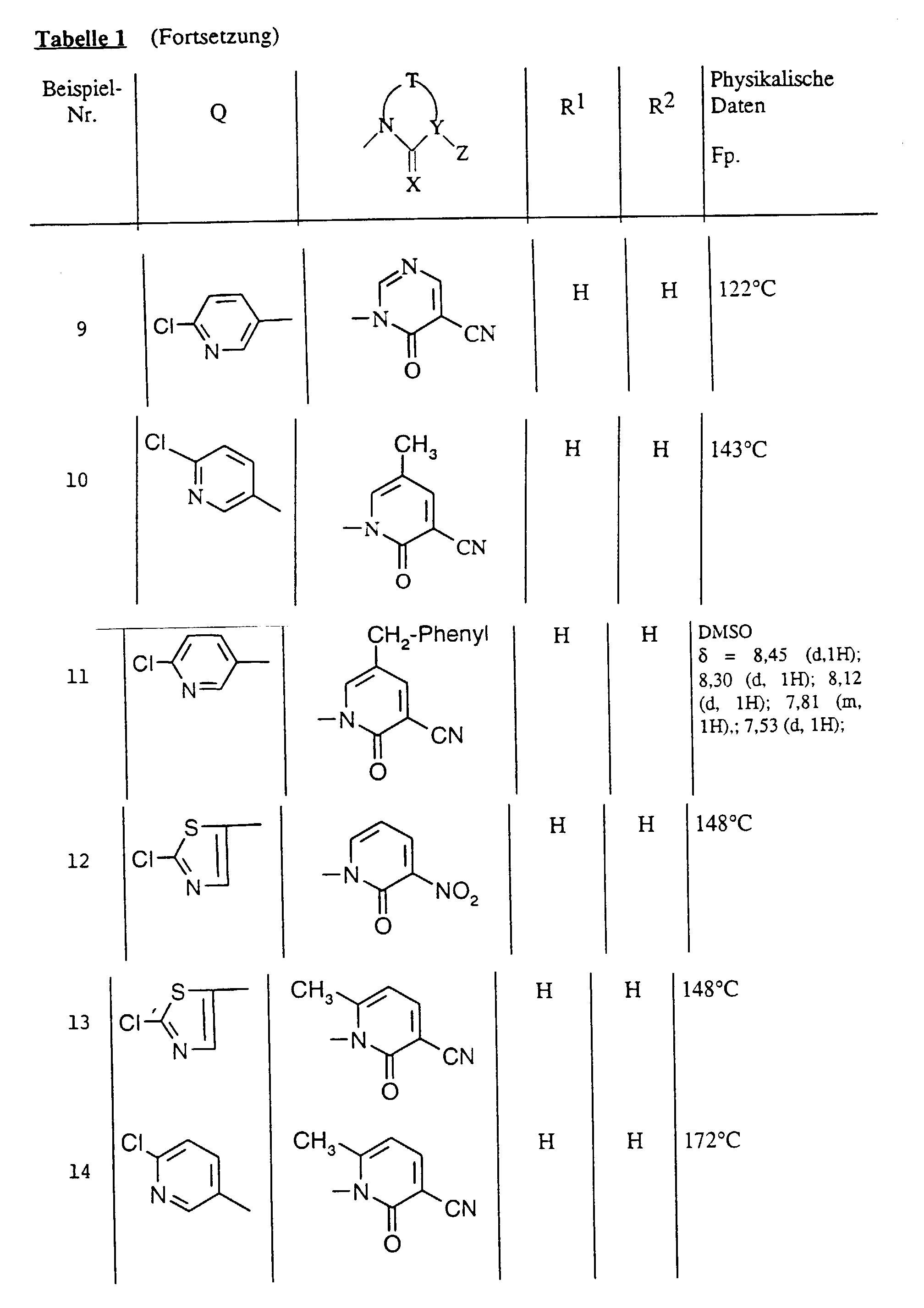

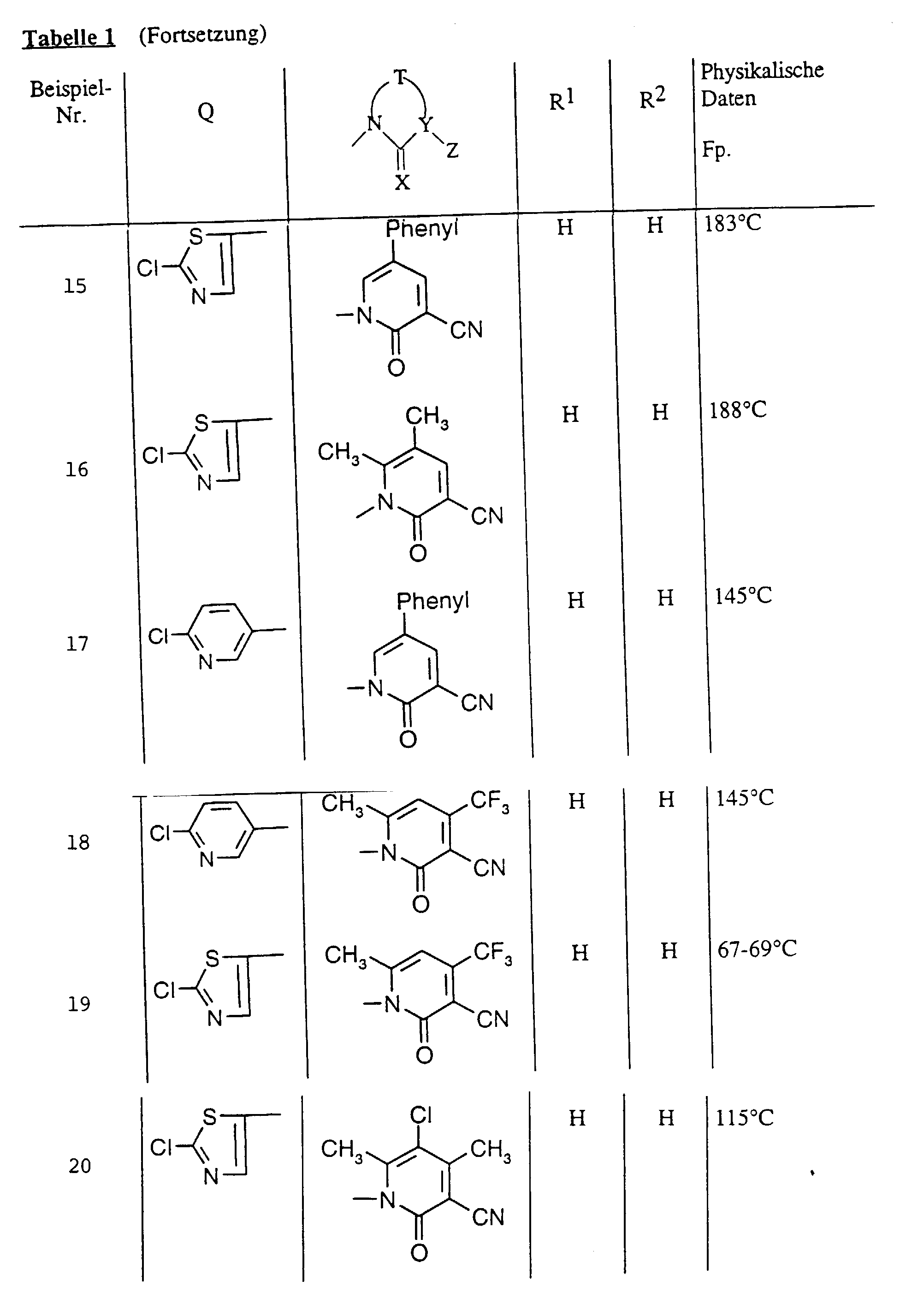

## Patentansprüche

1. Substituierte Stickstoff-Heterocyclen der allgemeinen Formel (I), 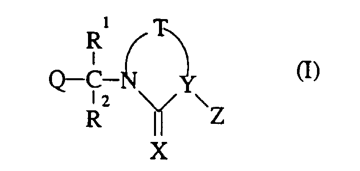 in welcher die Gruppierung 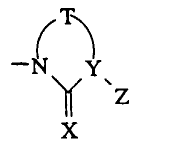 eine der folgenden Bedeutungen 1 bis 14 annimmt: 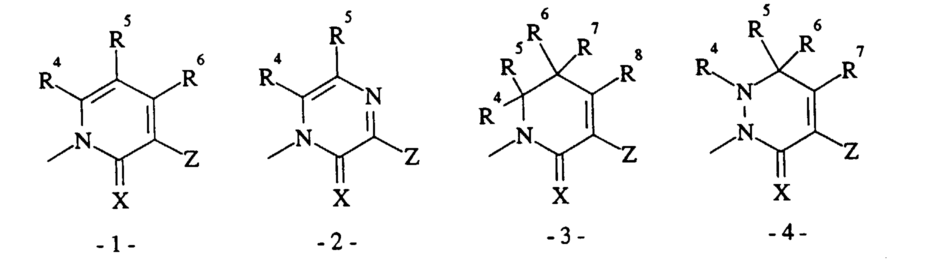 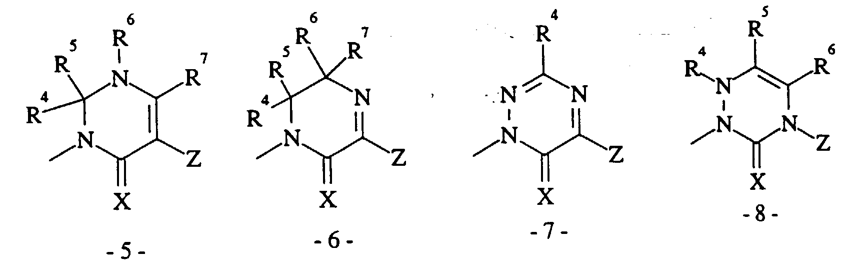 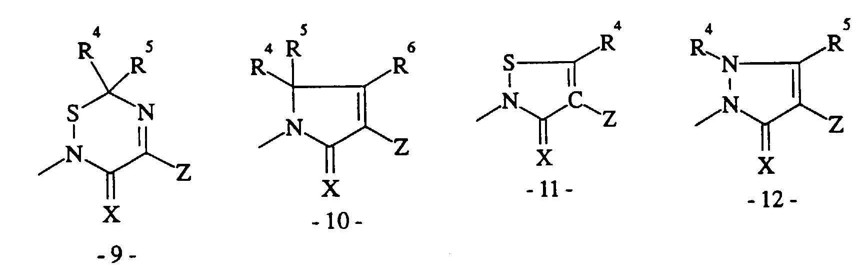 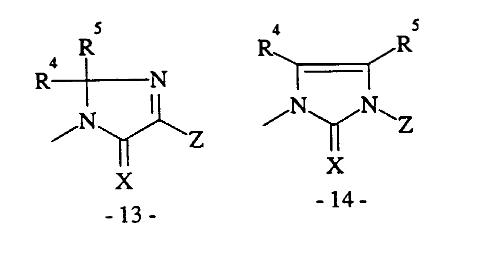 wobei
R¹ für Wasserstoff, Methyl oder Ethyl steht,
R² für Wasserstoff steht,
X für Sauerstoff, Schwefel oder NR³ steht,
Z für eine Cyano- oder Nitrogruppe steht,
Q eine der folgenden Bedeutungen annimmt: 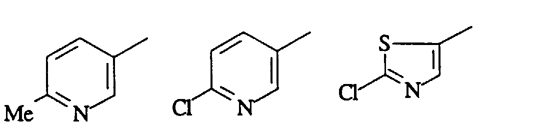 in welcher
R³ für Wasserstoff oder Methyl steht,
R⁴, R⁵, R⁶, R⁷ und R⁸ unabhängig voneinander für Wasserstoff, Fluor, Chlor, Brom, C₁₋₂-Alkyl, C₁₋₂-Alkoxy und gegebenenfalls durch Fluor, Chlor, Brom, C₁₋₂-Alkyl, Trifluormethyl, C₁₋₂-Alkoxy, Trifluormethoxy ein- oder mehrfach substituiertes Phenyl steht.

2. Verfahren zur Herstellung der substituierten Stickstoff-Heterocyclen der allgemeinen Formel (I) 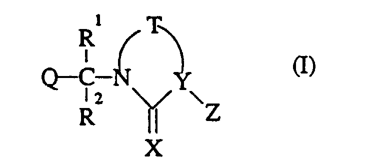 in welcher die Reste die in Anspruch 1 angegebene Bedeutung haben
dadurch gekennzeichnet, daß man
Stickstoff-Heterocyclen der Formel (II) 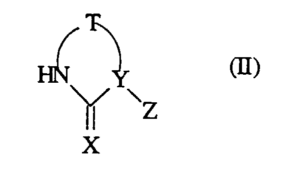 in welcher
die Reste die oben angegebene Bedeutung haben,
mit Halogenmethyl-Verbindungen der Formel (III) 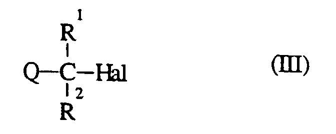 in welcher
Hal für Chlor, Brom oder Iod steht, und Q, R¹ und R² die oben angegebene Bedeutung haben,
gegebenenfalls in Gegenwart eines Verdünnungsmittels und in Gegenwart einer Base umsetzt.

3. Schädlingsbekämpfungsmittel, gekennzeichnet durch einen Gehalt an mindestens einem substituierten Stickstoffheterocyclen der Formel (I), gemäß Anspruch 1.

4. Verwendung von substituierten Stickstoffheterocyclen der Formel (I) gemäß Anspruch 1, zur Bekämpfung von Schädlingen.

5. Verfahren zur Bekämpfung von Schädlingen, dadurch gekennzeichnet, daß man substituierte Stickstoffheterocyclen der Formel (I) gemäß Anspruch 1 auf Schädlinge und/oder ihren Lebensraum einwirken läßt.

6. Verfahren zur Herstellung von Schädlingsbekämpfungsmitteln, dadurch gekennzeichnet, daß man substituierte Stickstoffheterocyclen der Formel (I) gemäß Anspruch 1 mit Streckmitteln und/oder oberflächenaktiven Mitteln vermischt

7. Verwendung von substituierten Stickstoffheterocyclen der Formel (I) gemäß Anspruch 1, zur Herstellung von Schädlingsbekämpfungsmitteln.

## Claims

1. Substituted nitrogen heterocycles of the general formula (I) 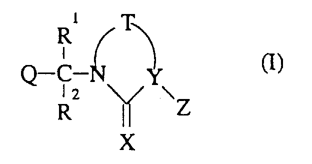 in which the group 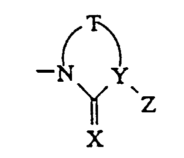 assumes one of the following meanings 1 to 14: 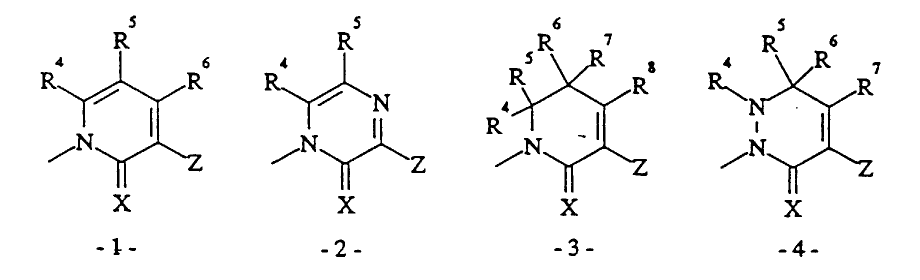 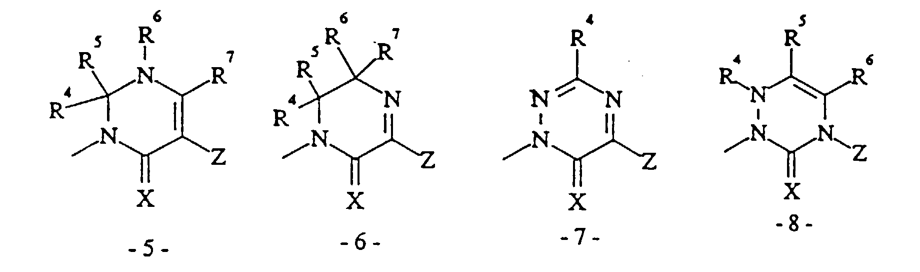 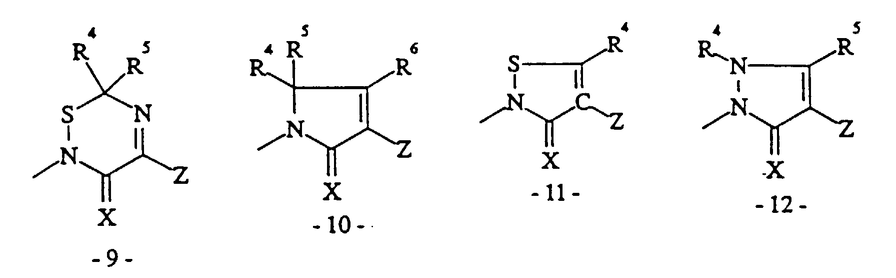 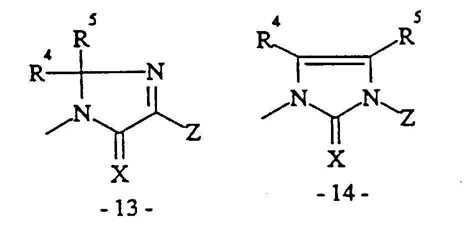 in which
R¹ represents hydrogen, methyl or ethyl,
R² represents hydrogen,
X represents oxygen, sulphur or NR³,
Z represents a cyano or nitro group,
Q assumes one of the following meanings: 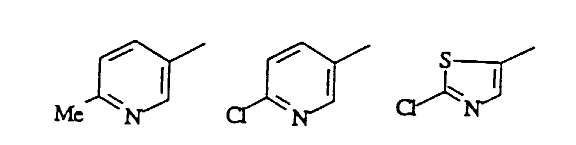 in which
R³ represents hydrogen or methyl, and
R⁴, R⁵, R⁶, R⁷ and R⁸ independently of one another represent hydrogen, fluorine, chlorine, bromine, C₁₋₂alkyl, C₁₋₂-alkoxy, and phenyl which is optionally mono- or polysubstituted by fluorine, chlorine, bromine, C₁₋₂-alkyl, trifluoromethyl, C₁₋₂-alkoxy or trifluoromethoxy.

2. Process for the preparation of the substituted nitrogen heterocycles of the general formula (I) 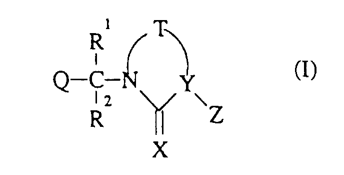 in which the radicals have the meaning given in Claim 1
characterized in that
nitrogen heterocycles of the formula (II) 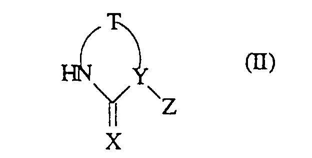 in which
the radicals have the abovementioned meaning are reacted with halogenomethyl compounds of the formula (III) 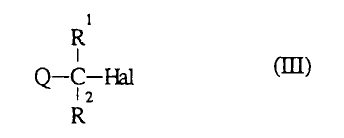 in which
Hal represents chlorine, bromine or iodine and Q, R¹ and R² have the abovementioned meaning,
if appropriate in the presence of a diluent and in the presence of a base.

3. Pesticides, characterized in that they contain at least one substituted nitrogen heterocycle of the formula (I) according to Claim 1.

4. Use of substituted nitrogen heterocycles of the formula (I) according to Claim 1 for combating pests.

5. Method of combating pests, characterized in that substituted nitrogen heterocycles of the formula (I) according to Claim 1 are allowed to act on pests and/or their environment.

6. Process for the preparation of pesticides, characterized in that substituted nitrogen heterocycles of the formula (I) according to Claim 1 are mixed with extenders and/or surface-active agents.

7. Use of substituted nitrogen heterocycles of the formula (I) according to Claim 1 for the preparation of pesticides.

## Revendications

1. Composés hétérocycliques azotés substitués répondant à la formule générale (I) 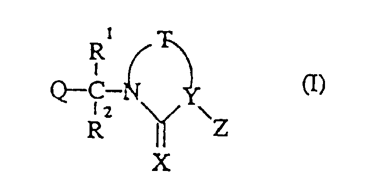 dans laquelle le groupement 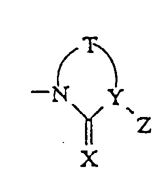 prend une des significations 1 à 14 ci-après: 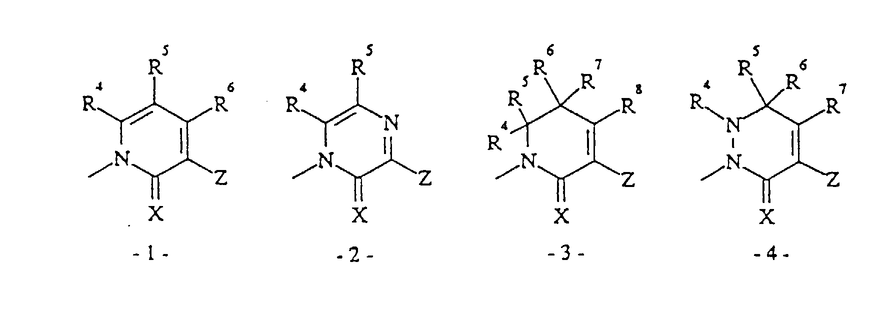 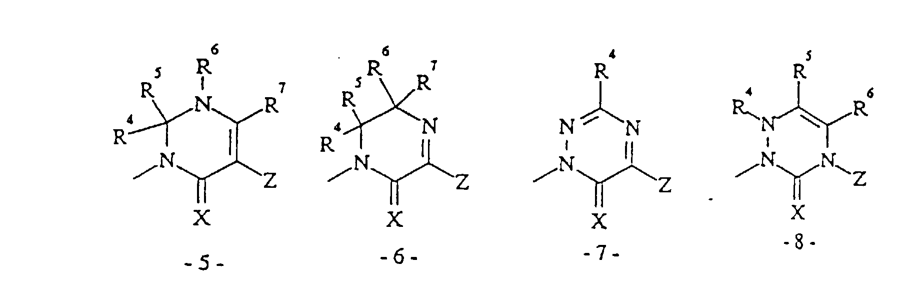 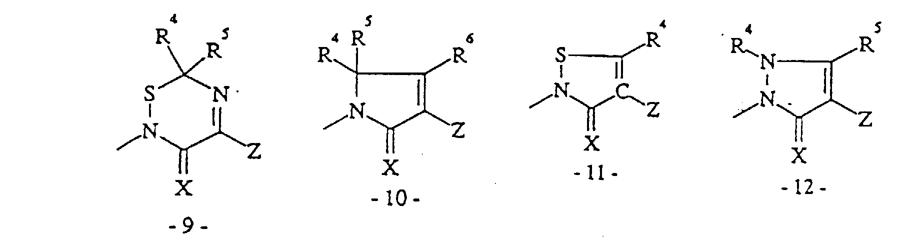 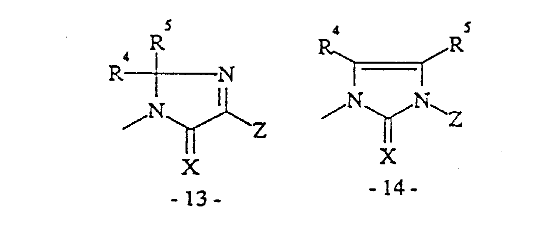 dans lesquelles
R¹ représente un atome d'hydrogène, un groupe méthyle ou un groupe éthyle,
R² représente un atome d'hydrogène,
X représente un atome d'oxygène, un atome de soufre ou NR³,
Z représente un groupe cyano ou nitro,
Q prend une des significations ci-après: 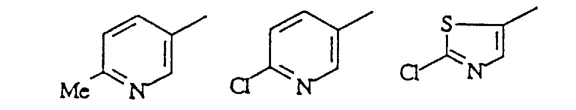
R³ représentant un atome d'hydrogène ou un groupe méthyle,
R⁴, R⁵, R⁶, R⁷ et R⁸ représentent, indépendamment l'un de l'autre, un atome d'hydrogène, un atome de fluor, un atome de chlore, un atome de brome, un groupe alkyle en C₁-C₂, un groupe alcoxy en C₁-C₂ et un groupe phényle portant éventuellement un ou plusieurs substituants identiques ou différents fluoro, chloro, bromo, alkyle en C₁-C₂, trifluorométhyle, alcoxy en C₁-C₂, trifluorométhoxy.

2. Procédé pour la préparation des composés hétérocycliques azotés substitués répondant à la formule générale (I) 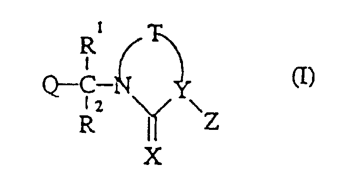 dans laquelle les radicaux ont la signification indiquée à la revendication 1,
caractérisé en ce qu'on fait réagir
des composés hétérocycliques azotés de formule (II) 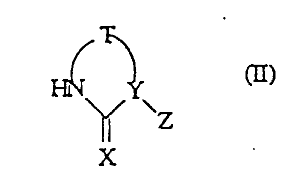 dans laquelle les radicaux ont la signification indiquée ci-dessus,
avec des composés d'halogénométhyle de formule (III) 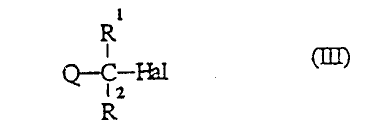 dans laquelle
Hal représente un atome de chlore, un atome de brome ou un atome d'iode et Q, R¹ et R² ont la signification indiquée ci-dessus,
éventuellement en présence d'un diluant et en présence d'une base.

3. Agent de lutte contre les parasites, caractérisé par une teneur en au moins un composé hétérocyclique azoté substitué de formule (I) selon la revendication 1.

4. Utilisation de composés hétérocycliques azotés substitués de formule (I) selon la revendication 1, pour lutter contre des parasites.

5. Procédé pour lutter contre des parasites, caractérisé en ce qu'on laisse agir des composés hétérocycliques azotés substitués de formule (I) selon la revendication 1 sur des parasites et/ou sur leur biotope.

6. Procédé pour la préparation d'agents de lutte contre les parasites, caractérisé en ce qu'on mélange des composés hétérocycliques azotés substitués de formule (I) selon la revendication 1 avec des diluants et/ou des agents tensioactifs.

7. Utilisation de composés hétérocycliques azotés substitués de formule (I) selon la revendication 1, pour la préparation d'agents de lutte contre les parasites.
